(19) Europäisches Patentamt
European Patent Office
Office européen des brevets

(11) Numéro de publication : **0 507 674 A1**

# (12) DEMANDE DE BREVET EUROPEEN

(21) Numéro de dépôt : **92400905.3**

(22) Date de dépôt : **01.04.92**

(51) Int. Cl.$^5$ : **C07C 45/27,** C07C 45/51, C07C 45/56, C07C 209/68

(30) Priorité : **02.04.91 FR 9103946**

(43) Date de publication de la demande : **07.10.92 Bulletin 92/41**

(84) Etats contractants désignés : **AT BE CH DE DK ES FR GB GR IT LI LU NL PT SE**

(71) Demandeur : **RHONE-POULENC NUTRITION ANIMALE**
**Rue Marcel Lingot**
**F-03600 Commentry (FR)**

(72) Inventeur : **Chabardes, Pierre**
**24 rue Jeanne d'Arc**
**F-69110 Sainte Foy Les Lyon (FR)**
Inventeur : **Henrot, Serge**
**Les Pres de la Riviere Plate**
**F-69440 Sainte Catherine (FR)**

(74) Mandataire : **Le Pennec, Magali et al**
**RHONE-POULENC RORER SA, Direction des Brevets, 20 Avenue Raymond Aron**
**F-92160 Antony (FR)**

## (54) Procédé de préparation d'aldéhydes insaturés.

(57) L'invention concerne un procédé de préparation d'aldéhydes insaturés par oxydation d'amines tertiaires allyliques en présence d'un hydroperoxyde d'alkyle et d'un catalyseur à base de vanadium.

EP 0 507 674 A1

Jouve, 18, rue Saint-Denis, 75001 PARIS

La présente invention concerne un procédé de synthèse d'aldéhydes insaturés. Plus particulièrement, elle concerne un procédé de synthèse d'aldéhydes insaturés à partir d'amines tertiaires allyliques.

Il est connu dans l'art antérieur de préparer des N-oxydes d'amines tertiaires par oxydation des amines correspondantes en présence d'un hydroperoxyde et d'un catalyseur métallique. En particulier, Sheng et al (J. Org. Chem., 1968, 33, 588 ; Organic Synthesis 1970, 50, 56) décrit la préparation du N-oxyde de la tri-n-butylamine, de la triéthylamine et du 1-diméthylamino-2 propanol. Les métaux utilisés sont notamment le vanadium, le molybdène, le chrome, le cobalt, le fer et le manganèse ; l'hydroperoxyde étant choisi parmi les hydroperoxydes de cumyle et d'amylène.

Il est également connu de réaliser la N-déalkylation d'amines tertiaires en présence d'un catalyseur au ruthénium et d'hydroperoxyde de tertiobutyle (Murahashi et al., J. Am. Chem. Soc., 1988, 110, 8256). Ce procédé étant particulièrement adapté à la déméthylation sélective de méthylamines tertiaires.

Cependant, aucun de ces documents ne décrit ni ne suggère la possibilité d'oxyder directement les amines tertiaires en aldéhydes. A fortiori, rien dans ces documents ne suggère la possibilité d'oxyder directement des amines tertiaires allyliques dont la réactivité est différente des amines aliphatiques décrites.

La demanderesse a maintenant montré qu'il est possible d'oxyder les amines tertiaires allyliques, pour former les aldéhydes insaturés correspondants. Par ailleurs, la demanderesse a également montré que l'oxydation conduisait aussi à la formation de l'énamine correspondante.

L'objet de la présente invention réside donc dans un procédé de préparation d'un aldéhyde insaturé et/ou de son énamine caractérisé en ce que l'on oxyde l'amine tertiaire allylique correspondante en présence d'un hydroperoxyde d'alkyle et d'un catalyseur à base de vanadium.

L'aldéhyde et l'énamine formés se trouvent dans un certain équilibre que l'on déplace facilement vers l'aldéhyde lorsque l'on se place en milieu acide dilué.

Les amines tertiaires allyliques utilisables dans la présente invention répondent notamment à la formule générale $(R_1)(R_2)N(R_3)$ dans laquelle $R_1$, $R_2$ et $R_3$, égaux ou différents, sont choisis parmi les groupements alkyl C1 à C30 ou alkényl C2 à C30, linéaires, ramifiés, substitués ou non, cycliques ou contenant un cycle, ayant éventuellement un ou plusieurs hétéroatomes, et dans laquelle $R_1$ et $R_2$ peuvent former entre eux un cycle et $R_3$ au moins représente un groupement allylique.

Au sens de la présente invention, on entend par groupement allylique tout groupement ayant au moins une double liaison en $\beta,\gamma$ de l'atome d'azote. Préférentiellement, $R_1$ et $R_2$, égaux ou différents, sont des groupements alkyl, linéaires ou ramifiés, contenant 1 à 4 atomes de carbone et pouvant former entre eux un cycle ou un hétérocycle.

Encore plus préférentiellement, $R_1$ et $R_2$, égaux ou différents, représentent un groupement méthyl ou éthyl.

Préférentiellement, $R_3$ est un groupement allylique contenant entre 3 et 15 atomes de carbone.

A titre d'exemple d'amines tertiaires allyliques utilisables dans la présente invention, on peut citer notamment la N-dialkylprénylamine, la N-dialkylgéranylamine ou son isomère la N-dialkylnérylamine, ou encore la N-dialkyl (triméthyl-2,6,6 cyclohexène-1) méthanamine.

Dans ce qui suit, le terme géranylamine désigne le mélange des deux isomères géranyl et nérylamine. On préfère utiliser la diéthylgéranylamine.

Les catalyseurs à base de vanadium utilisables dans la présente invention sont notamment des sels du vanadium, à tous les degrés d'oxydation possibles. En particulier, dans les degrés d'oxydation élevés, on peut citer $V_2O_5$, $NH_4VO_3$, les vanadates d'alcoyle tels que l'orthovanadate de triéthanolamine (OVTEA) et l'orthovanadate d'octadécyle $[OV(OC_{18}H_{37})_3]$, l'acétylacétonate de vanadyle $[OV(AcAc)_2]$, le sulfate de vanadyle $(OVSO_4)$, ou l'orthovanadate de méthylcyclohexanol.

Selon la présente invention, il est possible d'utiliser simultanément plusieurs catalyseurs à base de vanadium.

Par ailleurs, ces différents catalyseurs peuvent être utilisés en suspension ou supportés. Dans ce dernier cas, on peut utiliser les supports de type oxyde, tels que notamment les alumines ou les silices. On peut également utiliser les charbons ou encore les résines.

Le procédé de l'invention est généralement mis en oeuvre en présence de quantités catalytiques de vanadium. Il est entendu que l'homme de l'art peut adapter cette quantité en fonction de la vitesse de réaction recherchée, de l'amine et de l'hydroperoxyde utilisés. Préférentiellement, le rapport molaire catalyseur à base de vanadium (compté en métal)/amine est compris envi 0,0001 et 0,5.

Selon la présente invention, on utilise préférentiellement un hydroperoxyde d'alkyle de formule:

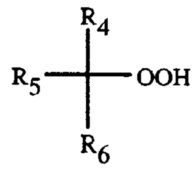

dans laquelle $R_4$, $R_5$ et $R_6$, égaux ou différents, représentent :

- des atomes d'hydrogène,
- des groupements alkyle linéaires ou ramifiés comportant de 1 à 3 atomes de carbone,
- des groupements cycloalkyle comportant de 3 à 12 atomes de carbone, ou
- des groupements alkyl- ou cycloalkylaromatiques comportant de 7 à 30 atomes de carbone.

Dans un mode préféré de mise en oeuvre, l'hydroperoxyde est choisi parmi les hydroperoxydes de cumyle, d'amylène, de tertiobutyle ou de cyclohexyle.

Le procédé de l'invention est avantageusement mis en oeuvre dans un solvant. En particulier, le solvant peut être choisi parmi :

- les hydrocarbures (pentane, hexane ...),
- Les éthers (méthylterbutyléther),
- les esters (acétate d'éthyle),
- les alcools (méthanol ...),
- Les solvants aromatiques (toluène, xylène, benzène), ou
- les solvants halogénés (chlorure de méthylène, chlorobenzène).

La réaction est avantageusement conduite à une température comprise entre la température ambiante et 80°C environ. La température est notamment adaptée en fonction du solvant utilisé.

Pour une bonne mise en oeuvre de l'invention, il est généralement préférable de travailler en présence d'un excès d'oxydant par rapport à l'amine. Cet excès ne doit toutefois pas être trop important pour éviter l'oxydation des aldéhydes insaturés formés. Dans un mode préféré de mise en oeuvre, le rapport molaire hydroperoxyde/amine est compris entre 0, 1 et 5.

Comme indiqué ci-avant, le produit de la réaction comprend généralement un mélange de l'aldéhyde et de son énamine. Ces deux composés peuvent être séparés et utilisés comme tels. Toutefois, l'énamine formée peut être aisément convertie en aldéhyde par hydrolyse notamment en milieu acide dilué. Cette conversion est quantitative et très rapide, ainsi qu'illustré dans les exemples. Préférentiellement, cette réaction est réalisée dans des zones de pH proches de 4. A titre d'exemple, l'acide acétique donne de très bons résultats.

Dans un mode particulier de mise en oeuvre de l'invention, le procédé est conduit en présence d'un agent desséchant. Il peut s'agir en particulier de tamis moléculaire 4Å, de sulfate de magnésium ou de sulfate de sodium. Comme le montrent les exemples, la présence d'un tel agent a permis d'améliorer les rendements de la réaction.

Le procédé de l'invention est particulièrement adapté à la préparation de prénal et de citral, qui constituent des intermédiaires importants en synthèse organique, en particulier dans la synthèse des vitamines A et E. Dans le cas du citral, la réaction peut être schématisée de la manière suivante:

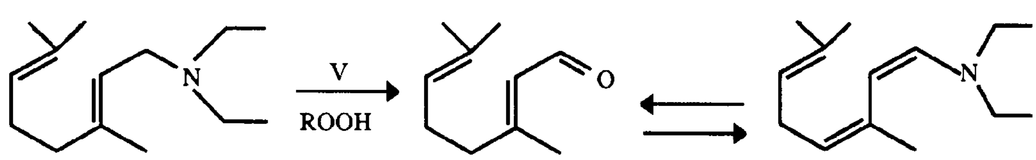


DEGA Citral Enamine

L'invention sera plus complètement décrite à l'aide des exemples suivants qui doivent être considérés comme illustratifs et non limitatifs.

Dans les exemples 1 à 5, les rendements en citral indiqués représentent le citral directement obtenu plus le citral provenant de l'hydrolyse acide de l'énamine du citral.

## EXEMPLE 1

On place dans un réacteur, dans l'ordre suivant, 20 mg de $V_2O_5$, 420 mg de diéthylgéranylamine (DEGA), 100 mg d'un étalon interne $C_{11}$ et 5 ml (6,65 g) de chlorure de méthylène. 530 mg d'hydroperoxyde de tertiobutyle 3M dans l'isooctane sont ajoutés lentement en 15 minutes à température ambiante.

Après 3 heures de réaction à température comprise entre 27 et 29°C, le milieu est refroidi à 5°C, et 1 ml d'eau est ajouté. Les produits de réaction sont analysés par chromatographie en phase vapeur. Les résultats sont les suivants:

- taux de transformation de la DEGA : 93 %
- rendement en citral : 50 % (dont 5 % d'énamine avant l'addition de 1 ml d'eau

EXAMPLE 2

On procède comme dans l'exemple 1, mais en utilisant comme catalyseur $OVSo_4$-$5H_2O$ à la place de $W_2O_5$. On obtient les résultats suivants :

- taux de transformation de la DEGA : 76 %
- rendement en citral : 15 % (dont 4 % d'énamine avant l'addition de 1 ml d'eau).

EXEMPLE

Dans un ballon, on place 420 mg (2 mmoles) de DEGA, 5 ml de chlorure de méthylène, 0,2 mmole d'orthovanadate de méthylcyclohexanol, et un étalon interne ($C_{11}$). 2 mmoles d'hydroperoxyde de tertiobutyle 3M dans l'isooctane sont ajoutées lentement et la température est maintenue à 40°C.

Le citral formé est dosé en CPV. On obtient un rendement de 17 %.

EXAMPLE 4

Dans un ballon, on place 420 mg de DEGA, 5 ml de toluène, 0,2 mmole d'orthovanadate de triéthanolamine et un étalon interne ($C_{11}$). 2 mmoles d'hydroperoxyde de tertiobutyle 3M dans le toluène sont ajoutées lentement et la température est maintenue à 20°C.

Le citral formé est dosé en CPV. On obtient un rendement de 17 %.

EXEMPLE 5

On procède comme dans l'exemple 4, mais en présence de tamis moléculaire 4Å.

On obtient un rendement en citral de 42 %.

EXEMPLE 6

Cet exemple illustre l'hydrolyse de l'énamine en aldéhyde.

On utilise une solution contenant, dans le pentane :

- citral : 51 mg
- DEGA : 119 mg
- énamine du citral : 44 mg (préparée selon la publication de Mannich et Coll. dans Berichte 69, 2112 (1936).

Cette composition est stable sur au moins 96 heures. A cette solution est ajouté 10 % d'acide acétique et, au bout de 5 à 30 minutes à température ambiante, le citral présent est dosé par CPV avec étalonnage interne. On obtient 85 mg de citral pour une quantité théorique de 86 mg attendue.

**Revendications**

**1 -** Procédé de préparation d'un aldéhyde insaturé et/ou de son énamine caractérisé en ce que l'on oxyde l'amine tertiaire allylique correspondante en présence d'un hydroperoxyde d'alkyle et d'au moins un catalyseur à base de vanadium.

**2** - Procédé selon la revendication 1 caractérisé en ce que l'amine tertiaire allylique a pour formule générale $(R_1)(R_2)N(R_3)$ dans laquelle $R_1$, $R_2$ et $R_3$, égaux ou différents, sont choisis parmi les groupements alkyl C1 à C30 ou alkényl C2 à C30 linéaires, ramifiés, substitués ou non, éventuellement cycliques ou contenant un cycle, ayant éventuellement 1 ou plusieurs hétéroatomes, et dans laquelle $R_1$ et $R_2$ peuvent former entre eux un cycle et $R_3$ au moins représente un groupement allylique.

**3** - Procédé selon la revendication 2 caractérisé en ce que $R_1$ et $R_2$, égaux ou différents, sont des groupements alkyl linéaires ou ramifiés, contenant 1 à 4 atomes de carbone et pouvant former entre eux un cycle ou un hétérocycle.

**4** - Procédé selon la revendication 3 caractérisé en ce que $R_1$ et $R_2$, égaux ou différents, représentent un groupement méthyl ou éthyl.

**5** - Procédé selon la revendication 2 caractérisé en ce que $R_3$ est un groupement allylique contenant entre 3 et 15 atomes de carbone.

**6** - Procédé selon l'une quelconque des revendications 2 à 5 caractérisé en ce que l'amine est choisie parmi la N-dialkylprénylamine, la N-dialkylgéranylamine, la N-dialkylnérylamine et la N-dialkyl (triméthyl-2,6,6 cyclohexène-1) méthanamine.

**7** - Procédé selon la revendication 1 caractérisé en ce que l'hydroperoxyde d'alkyle répond à la formule générale :

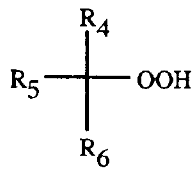

dans laquelle $R_4$, $R_5$ et $R_6$, égaux ou différents, sont choisis parmi :

- des atomes d'hydrogène,
- des groupements alkyl linéaires ou ramifiés comportant 1 à 3 atomes de carbone,
- des groupements cycloalkyle comportant de 3 à 12 atomes de carbone, et
- des groupements alkyl- ou cycloalkylaromatiques comportant de 7 à 30 atomes de carbone.

**8** - Procédé selon la revendication 1 caractérisé en ce que les catalyseurs à base de vanadium sont en suspension ou supportés.

**9** - Procédé selon la revendication 1 caractérisé en ce que l'on opère en présence d'un solvant choisi parmi les hydrocarbures, les éthers, les esters, les alcools, les solvants aromatiques et les solvants halogénés.

**10** - Procédé selon la revendication 1 caractérisé en ce que le rapport molaire hydroperoxyde d'alkyle/amine est compris entre 0, 1 et 5.

**11** - Procédé selon la revendication 1 caractérisé en ce que la température est comprise entre la température ambiante et 80°C environ.

**12** - Procédé selon la revendication 1 caractérisé en ce que l'on opère en outre en présence d'un agent desséchant, tel que le tamis moléculaire 4Å, le sulfate de magnésium ou de sodium.

**13** - Procédé selon la revendication 1 caractérisé en ce que l'on convertit l'énamine formée en aldéhyde insaturé par hydrolyse en milieu acide.

Office européen des brevets

# RAPPORT DE RECHERCHE EUROPEENNE

Numero de la demande

EP 92 40 0905

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| D,A | JOURNAL OF ORGANIC CHEMISTRY.<br>vol. 33, no. 2, 1968, EASTON US<br>pages 588 - 590;<br>M.N. SHENG ET AL.: 'Hydroperoxide Oxidations Catalyzed by Metals. II. The Oxidation of Tertiary Amines to Amine Oxides.'<br>* le document en entier *<br>--- | 1 | C07C45/27<br>C07C45/51<br>C07C45/56<br>C07C209/68 |
| A | JOURNAL OF THE AMERICAN CHEMICAL SOCIETY<br>vol. 98, no. 21, 1976, WASHINGTON DC US<br>pages 6702 - 6704;<br>R.V. HOFFMAN: 'The Oxidation of Amines with Sulfonyl Peroxides.'<br>* le document en entier *<br>--- | 1 | |
| A | CHEMISTRY LETTERS.<br>no. 12, 1982, TOKYO JP<br>pages 1987 - 1988;<br>K. TAKABE ET AL.: 'A Simple Conversion of N,N-Dimethyl-2-Alkenylamine to 2-Alkenal.'<br>* le document en entier *<br>----- | 1 | **DOMAINES TECHNIQUES RECHERCHES (Int. Cl.5 )**<br>C07C |

Le présent rapport a été établi pour toutes les revendications

| Lieu de la recherche | Date d'achèvement de la recherche | Examinateur |
|---|---|---|
| LA HAYE | 02 JUILLET 1992 | BONNEVALLE E.I. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)